# EUROPÄISCHE PATENTSCHRIFT

(11) **EP 1 411 841 B1**
(45) Veröffentlichungstag und Bekanntmachung des Hinweises auf die Patenterteilung: **17.01.2007**
(21) Anmeldenummer: 02781177.7
(22) Anmeldetag: 31.07.2002
(51) Int. Cl.: A61B 17/26

(54) **CHIRURGISCHES SCHÄLMESSER**
SURGICAL PEELING KNIFE
RABOT CHIRURGICAL

(30) Priorität: 03.08.2001 DE 10138235
(43) Veröffentlichungstag der Anmeldung: 28.04.2004
(73) Patentinhaber: Aesculap AG & Co. KG, 78532 Tuttlingen (DE)
(72) Erfinder: DWORSCHAK, Manfred, 78589 Dürbheim (DE); LUTZE, Theodor, 78582 Balgheim (DE); MORALES, Pedro, 78532 Tuttlingen-Nendingen (DE); WEISSHAUPT, Dieter, 78194 Immendingen (DE)
(74) Vertreter: Regelmann, Thomas
(86) Internationale Anmeldenummer: PCT/EP2002/008494
(87) Internationale Veröffentlichungsnummer: WO 2003/013362

(56) Entgegenhaltungen:
- US-A- 1 881 250
- US-A- 4 038 986
- US-A- 4 221 222
- US-A- 5 330 495
- US-B1- 6 193 716

## Beschreibung

Die Erfindung betrifft ein chirurgisches Schälmesser mit einem Haltegriff und einem am Haltegriff fixierten Klingenhalter, welcher eine metallische Klinge hält, die quer zu einer Längsrichtung des Haltegriffes orientiert ist, wobei der Klingenhalter so ausgebildet ist, daß die Klinge versetzt zu einer Längsebene des Haltegriffs angeordnet ist.

US-A-5 330 495 offenbart ein chirurgisches Schälmesser gemäß dem Oberbegriff des ersten Anspruchs.

Solche chirurgischen Schälmesser werden auch als Ringmesser bezeichnet. Sie werden beispielsweise bei der Entfernung von Rachenmandeln eingesetzt. Mit ihnen kann über den Klingenhalter mit der Klinge Gewebe abgelöst werden über eine Art von ziehendem Schnitt, wobei zuvor eine Oberseite des Klingenhalters dem Schneidegebiet zu orientiert wurde bzw. sogar an den Schneidebereich angelegt wurde.

Der Erfindung liegt die Aufgabe zugrunde, ein chirurgisches Schälmesser der eingangs genannten Art zu schaffen, welches eine erhöhte Funktionalität aufweist.

Diese Aufgabe wird bei dem chirurgischen Schälmesser der eingangs genannten Art erfindungsgemäß dadurch gelöst, daß die Klinge eine erste Elektrode bildet und daß beabstandet zur Klinge an den Klingenhalter eine zweite Elektrode angeordnet ist.

Durch entsprechende Strombeaufschlagung läßt sich dann eine Spannung zwischen den beiden Elektroden ausbilden und geschnittenes Gewebe läßt sich damit verschorfen. Dadurch lassen sich beispielsweise die sonst beim Schnitt entstehenden Blutungen verhindern.

Günstigerweise sind dabei die Klinge und die zweite Elektrode bezogen auf jeweilige Seitenflächen im wesentlichen parallel zueinander ausgerichtet. Dadurch wirkt entlang einer Schneidkante der Klinge (quer zu einer Längsrichtung des Haltegriffes) gegenüber der zweiten Elektrode im wesentlichen die gleiche Spannung, so daß über die Schneidkante hinweg auch bezüglich Verschorfung die gleichen Bedingungen vorliegen.

Vorteilhafterweise liegt dabei bezogen auf ein Ende des Klingenhalters die zweite Elektrode vor der Klinge. Gewebe wird mittels eines Schälmessers über eine Art ziehenden Schnitt geschnitten. Liegt die Elektrode vor der Klinge, dann wird zuerst ein Schnitt durchgeführt und gleichzeitig oder unmittelbar danach läßt sich das Gewebe verschorfen.

Günstigerweise ist die Klinge so angeordnet, daß eine Schneidkante von dem Handgriff weg orientiert ist. Auf diese Weise läßt sich ein ziehender Schnitt in ein Gewebe einbringen, um beispielsweise so Rachenmandeln oder dergleichen zu entfernen.

Eine Schneidkante der Klinge weist dabei einen größeren Höhenabstand zu dem Haltegriff auf als eine Kante der zweiten Elektrode. Insbesondere ist die zweite Elektrode im wesentlichen bündig mit einer Oberfläche des Klingenhalters angeordnet. Dadurch wird der Schneidvorgang durch die zweite Elektrode nicht behindert.

Vorteilhafterweise ist die Klinge so angeordnet, daß ihre Schneidkante unterhalb eines Scheitels des Klingenhalters in Richtung eines vorderen Endes des Klingenhalters sitzt. Dadurch läßt sich ein ziehender Schnitt auch durch eine Art Drehbewegung des Klingenhalters einbringen.

Bei einer Variante einer Ausführungsform umfaßt der Klingenhalter einen ersten Bügel und einen zweiten Bügel, zwischen welchen die Klinge und die zweite Elektrode angeordnet sind und zwischen welchen ein Zwischenraum gebildet ist. Über den Zwischenraum läßt sich die Klinge auf einen Klingenhalter aufsetzen. Über den Zwischenraum kann auch eine Absaugvorrichtung zum Absaugen von geschnittenem Gewebe an ein Schneidgebiet herangeführt werden.

Ganz besonders vorteilhaft ist es, wenn das Schälmesser aus einem isolierenden Kunststoffmaterial hergestellt ist. Dadurch ist bei entsprechender Anordnung der beiden Elektroden systematisch für eine Isolierung gesorgt; insbesondere lassen sich dann auch Zuführungsleitungen und insbesondere Pole für die Elektroden in das Kunststoffmaterial einbetten, wobei dann getrennte Pole elektrisch voneinander isoliert sind.

Vorteilhafterweise ist also eine elektrische Zuführung zu den Elektroden in den Haltegriff und/oder den Klingenhalter eingebettet.

Günstigerweise ist ein Hochfrequenz-Anschluß für die Elektroden vorgesehen, so daß das Schälmesser als bipolares Ringmesser einsetzbar ist, bei welchem ein Wechselstrom zwischen die Elektroden anlegbar ist.

Bei einer Variante einer Ausführungsform ist an dem Klingenhalter eine Aufnahme mit einem oder mehreren Formschlußelementen zum Fixieren der Klinge angeordnet. Beispielsweise kann es sich dabei um Bolzen oder Schraubverbindungen handeln. Es kann dazu alternativ oder zusätzlich vorgesehen sein, daß die Klinge mit der Klingenaufnahme verschweißt wird.

Zur Sicherstellung eines elektrischen Kontaktes zwischen der Klinge und dem zugeordneten Pol ist günstigerweise eine Klingenaufnahme mit einer elektrischen Zuführung zur Stromversorgung der Klinge verbunden. Insbesondere weist die Klingenaufnahme ein Kontaktelement für die Klinge auf, so daß ein elektrischer Kontakt gewährleistet ist.

Bei einer Variante einer Ausführungsform ist an dem Kontaktelement ein leitendes federndes Element angeordnet, welches in Richtung der Klinge vorgespannt ist. Das federnde Element preßt sich dadurch gegen die Klinge und sorgt für den elektrischen Kontakt zwischen der Kontaktaufnahme und der Klinge.

Es kann auch vorgesehen sein, daß das Kontaktelement fest mit der Klinge verbunden ist, beispielsweise über Formschluß, Schweißen oder Löten. Die elektrische Verbindung zwischen dem Kontaktelement und der Klinge wird bei dieser Ausführungsform dann durch die entsprechende Verbindung gesichert.

Die nachfolgende Beschreibung einer bevorzugten Ausführungsform dient im Zusammenhang mit der Zeichnung der näheren Beschreibung der Erfindung. Es zeigen:
- Figur 1: eine perspektivische Ansicht eines Ausführungsbeispiels eines erfindungsgemäßen Schälmessers;

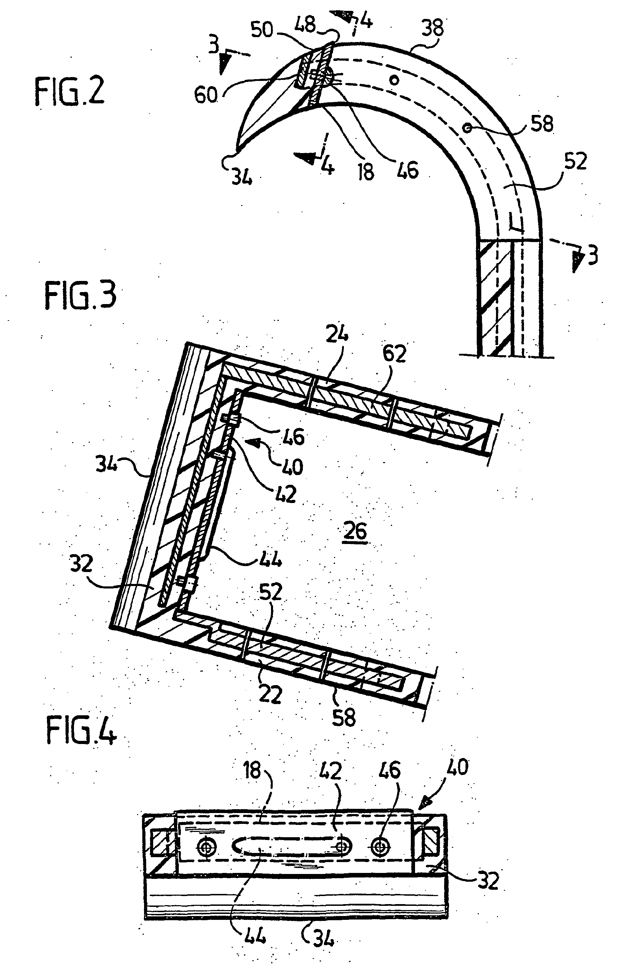

- Figur 2: eine Schnittansicht längs der Linie 2-2 gemäß Figur 1;
- Figur 3: eine Schnittansicht längs der Linie 3-3 gemäß Figur 2 und
- Figur 4: eine Schnittansicht längs der Linie 4-4 gemäß Figur 2.

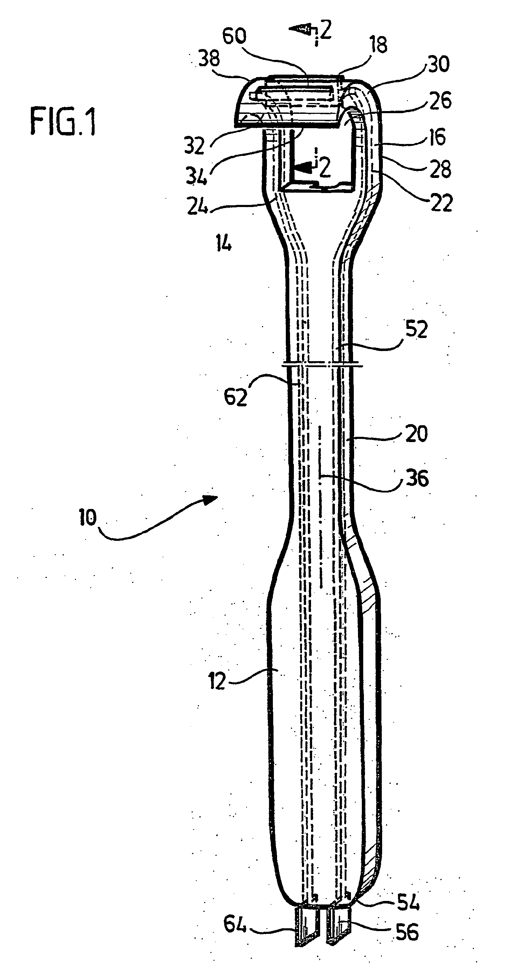

Ein Ausführungsbeispiel eines erfindungsgemäßen Schälmessers, welches in Figur 1 als Ganzes mit 10 bezeichnet ist, umfaßt einen Haltegriff 12 zum Halten des Schälmessers durch einen Operateur. An dem Haltegriff 12 ist ein Klingenhalter 14 angeordnet und insbesondere einstückig mit dem Haltegriff 12 verbunden. Der Klingenhalter 14 umfaßt dabei einen Haltekopf 16, welcher eine Klinge 18 hält, und ein Verbindungsteil 20, über welches der Haltekopf 16 mit dem Haltegriff 12 verbunden ist.

Der Haltegriff 12 und der Klingenhalter 14 sind vorzugsweise aus einem Kunststoffmaterial hergestellt.

Bei dem in Figur 1 gezeigten Ausführungsbeispiel ist der Haltegriff 12 gegenüber dem Verbindungsteil 20 verbreitert, so daß ein Operateur das Schälmesser 10 besser in der Hand halten kann. Zur Bereitstellung einer entsprechend großen Spannweite für die Klinge 18 ist wiederum der Haltekopf 16 gegenüber dem Verbindungsteil 20 verbreitert.

Der Haltekopf 16 umfaßt einen ersten Bügel 22 und einen zweiten Bügel 24, die jeweils beabstandet zueinander an dem Verbindungsteil 20 angeordnet sind. Zwischen den Bügeln 20 und 24 ist dabei ein materialfreier Zwischenraum 26 gebildet.

Die Bügel 22 und 24 weisen jeweils ein im wesentlichen gerades Teil 28 auf, an das sich ein Bogenteil 30 anschließt. Die vorderen Enden der Bogenteile 30 des ersten Bügels 22 und es zweiten Bügels 24 sind dabei über eine Leiste 32 miteinander verbunden, so daß ein vorderes Ende 34 der Leiste 32 ein vorderes Ende des Klingenhalters 14 bildet. Dieses vordere Ende liegt dabei bezogen auf eine Höhenabstandsrichtung 36 vom Haltegriff 12 unterhalb eines Scheitels 38 des Haltekopfs 16.

Des weiteren ist das vordere Ende 34 versetzt gegenüber einer Längsebene durch den Haltegriff 12, das heißt das vordere Ende 34 weist einen Abstand quer zur Längsrichtung 36 zu dem Haltegriff 12 auf.

Die Klinge 18 ist an der Leiste 32 angeordnet. Dazu weist diese eine Klingenaufnahme 40 auf. Diese umfaßt ein elektrisches Kontaktelement 42 in der Form einer Kontaktplatte.

Diese erstreckt sich insbesondere in dem Zwischenraum 26 zwischen einer Innenseite des ersten Bügels 22 und des zweiten Bügels 24 und ist dabei an der Leiste 32 angeordnet. An dem Kontaktelement 42 sitzt ein federndes Element 44, welches elektrisch leitend ist. Dieses federnde Element ist von dem Kontaktelement 42 weg vorgespannt, so daß beim Aufsetzen der Klinge 18 auf die Klingenaufnahme 40 das federnde Element 44 zwischen dem Kontaktelement 42 und der Klinge 18 liegt und eine Kraft auf die Klinge 18 ausübt. Auf diese Weise wird ein elektrischer Kontakt zwischen der Klinge 18 und dem Kontaktelement 42 sichergestellt.

Die Klinge 18 ist über Formschlußverbindungen wie beispielsweise Bolzenverbindungen 46 oder dergleichen an der Klingenaufnahme 40 fixierbar. Die Klinge 18 läßt sich dabei über den Zwischenraum 26 auf die Klingenaufnahme 40 aufsetzen und entsprechend sich über beispielsweise Bolzenverbindungen 46 dann dort fixieren (Figur 4).

Eine auf die Klingenaufnahme 40 aufgesetzte Klinge 18 ist so orientiert, daß ihre Schneidkante 48 auf einer Oberseite 50 des Klingenhalters 14, welche dem Haltegriff 12 abgewandt ist, herausragt. Die Schneidkante 48 ist dabei bezogen auf den Abstand zu dem Haltegriff in der Richtung 36 unterhalb des Scheitels 38 positioniert und in einer Querrichtung zu der Richtung 36 an der Leiste 32 zum vorderen Ende 34 hin positioniert.

Bei dieser Anordnung läßt sich Gewebematerial mittels des Schälmessers 10 durch eine entsprechende Bewegung abschälen; beispielsweise lassen sich so Rachenmandeln entfernen.

Die Klinge 18 ist dabei als eine erste Elektrode gebildet, welche über das Kontaktelement 42 und das federnde Element 44 mit einer elektrischen Zuführungsleitung 52 in Wirkverbindung steht.

Bei einer Variante einer Ausführungsform kann es noch alternativ oder zusätzlich vorgesehen sein, daß die Klinge 18 mit dem Kontaktelement 42 verschweißt ist.

Bei der Zuführungsleitung 52 handelt es sich dabei insbesondere um einen Metallpol, welcher durch den Klingenhalter 14 mit seinem Verbindungsteil 20 und durch den Haltegriff 12 hindurchgeführt ist. An einem unteren Ende 54 des Haltegriffs ist dann ein Anschlußelement 56 vorgesehen, über den Strom in die Zuführungsleitung 52 einspeisbar ist. Insbesondere läßt sich dabei ein Hochfrequenzstrom einspeisen.

Die Zuführungsleitung 52 ist in ein isolierendes Material des Haltegriffs 12 und des Klingenhalters 14 eingebettet. Beispielsweise wird bei der Herstellung des Haltegriffs 12 und des Klingenhalters 14, welche insbesondere einstückig miteinander verbunden sind, der Metallpol 52 in eine Spritzgußform eingelegt und dann umspritzt. Mittels Positionierstiften kann er dabei in der Form festgehalten werden. Bei dieser Variante einer Ausführungsform weist dann das erfindungsgemäße Schälmesser 10 Bohrungen 58 für Positionierstifte auf, welche entsprechend bei der Herstellung zur Fixierung der Zuführungsleitung 52 beispielsweise in einer Spritzgußform gedient haben.

Beabstandet zur Klinge 18 ist in der Leiste 32 eine zweite Elektrode 60 angeordnet, welche insbesondere in der Form einer Metallplatte ausgebildet ist. Diese zweite Elektrode 60 ist dabei im wesentlichen parallel zu der Klinge 18 bezogen auf eine Seitenfläche der Klinge 18 bzw. zweiten Elektrode orientiert. Sie ist so angeordnet und ausgebildet, daß sie im wesentlichen nicht über die Oberseite 50 des Klingenhalters 14 hinausragt. Sie ist mit einer Zuführungsleitung 62 als zweiten Pol verbunden, welche ebenfalls wie die Zuführungsleitung 52 durch den Klingenhalter 14 und den Haltegriff 12 geführt ist. An dem unteren Ende 54 ist wiederum ein Anschlußelement 64 vorgesehen, so daß sich ein Strom in das Schälmesser 10 einkoppeln läßt.

Über das Material des Haltegriffs 12 und des Klingenhalters 14, bei dem es sich insbesondere um ein Kunststoffmaterial wie PEEK oder LCP handelt, sind die beiden Elektroden 60 und 18 voneinander isoliert und die Zuführungsleitungen 52 und 62 sind ebenfalls voneinander isoliert. Es läßt sich dann eine Spannung zwischen einem oberen Ende der zweiten Elektrode 60 und der Klinge 18 erzeugen. Gleichzeitig oder unmittelbar nach dem Schneiden von Gewebe läßt sich dieses dann verschorfen, um beispielsweise Blutungen zu verhindern oder zu stoppen.

Es ist insbesondere vorgesehen, daß über die Anschlußelemente 56 und 64 ein Hochfrequenzstrom eingekoppelt wird. Das Schälmesser ist dann ein bipolares Schälmesser, bei dem entsprechend der Frequenz des eingekoppelten Stroms der erste Pol 52 und der zweite Pol 62 und damit wiederum die Klinge 18 und die zweite Elektrode 60 alternierend als Minuspol und Pluspol wirken.

## Patentansprüche

1. Chirurgisches Schälmesser mit einem Haltegriff (12) und einem am Haltegriff (12) fixierten Klingenhalter (14), welcher eine metallische Klinge (18) hält, die quer zu einer Längsrichtung (36) des Haltegriffs (12) orientiert ist, wobei der Klingenhalter (14) so ausgebildet ist, daß die Klinge (18) versetzt zu einer Längsebene des Haltegriffs (12) angeordnet ist,
**dadurch gekennzeichnet, daß** die Klinge (18) eine erste Elektrode bildet und daß beabstandet zur Klinge (18) an dem Klingenhalter (14) eine zweite Elektrode (60) angeordnet ist.

2. Chirurgisches Schälmesser nach Anspruch 1, **dadurch gekennzeichnet, daß** die Klinge (18) und die zweite Elektrode (60) bezogen auf jeweilige Seitenflächen im wesentlichen parallel zueinander ausgerichtet sind.

3. Chirurgisches Schälmesser nach Anspruch 1 oder 2, **dadurch gekennzeichnet, daß** bezogen auf ein Ende (34) des Klingenhalters (14) die zweite Elektrode (60) vor der Klinge (18) liegt.

4. Chirurgisches Schälmesser nach einem der vorangehenden Ansprüche, **dadurch gekennzeichnet, daß** die Klinge (18) so angeordnet ist, daß eine Schneidkante (48) von dem Haltegriff (12) weg orientiert ist.

5. Chirurgisches Schälmesser nach einem der vorangehenden Ansprüche, **dadurch gekennzeichnet, daß** eine Schneidkante (48) der Klinge (18) einen größeren Höhenabstand zu dem Haltegriff (12) aufweist als eine obere Kante der zweiten Elektrode (60).

6. Chirurgisches Schälmesser nach einem der vorangehenden Ansprüche, **dadurch gekennzeichnet, daß** die Klinge (18) so ausgebildet ist, daß ihre Schneidkante (48) unterhalb eines Scheitels (38) des Klingenhalters (14) in Richtung eines vorderen Endes (34) des Klingenhalters (14) sitzt.

7. Chirurgisches Schälmesser nach einem der vorangehenden Ansprüche, **dadurch gekennzeichnet, daß** der Klingenhalter (14) einen ersten Bügel (22) und einen zweiten Bügel (24) umfaßt, zwischen welchen die Klinge (18) und die zweite Elektrode (60) angeordnet sind und zwischen welchen ein Zwischenraum (26) gebildet ist.

8. Chirurgisches Schälmesser nach einem der vorangehenden Ansprüche, **dadurch gekennzeichnet, daß** das Schälmesser (10) im wesentlichen aus einem Kunststoffmaterial hergestellt ist.

9. Chirurgisches Schälmesser nach einem der vorangehenden Ansprüche, **dadurch gekennzeichnet, daß** eine elektrische Zuführung (52, 62) zu den Elektroden (18, 60) in den Haltegriff (12) und/oder den Klingenhalter (14) eingebettet ist.

10. Chirurgisches Schälmesser nach einem der vorangehenden Ansprüche, **gekennzeichnet durch** einen Hochfrequenz-Anschluß (56, 64) für die Elektroden (18, 60).

11. Chirurgisches Schälmesser nach einem der vorangehenden Ansprüche, **dadurch gekennzeichnet, daß** an dem Klingenhalter (14) eine Klingenaufnahme (40) mit einem oder mehreren Formschlußelementen zur Fixierung der Klinge (18) angeordnet ist.

12. Chirurgisches Schälmesser nach einem der vorangehenden Ansprüche, **dadurch gekennzeichnet, daß** eine Klingenaufnahme (40) mit einer elektrischen Zuführung (52) zur Stromversorgung der Klinge (18) verbunden ist.

13. Chirurgisches Schälmesser nach Anspruch 12, **dadurch gekennzeichnet, daß** die Klingenaufnahme (40) ein Kontaktelement (42) für die Klinge (18) aufweist.

14. Chirurgisches Schälmesser nach Anspruch 13, **dadurch gekennzeichnet, daß** an dem Kontaktelement (42) ein leitendes federndes Element (44) angeordnet ist, welches in Richtung der Klinge (18) vorgespannt ist.

15. Chirurgisches Schälmesser nach Anspruch 13, **dadurch gekennzeichnet, daß** das Kontaktelement fest mit der Klinge (18) verbunden ist.

## Claims

1. Surgical peeling knife having a handle (12) and a blade holder (14) which is secured to the handle (12) and holds a metallic blade (18) orientated transversely in relation to a longitudinal direction (36) of the handle (12), the blade holder (14) being so designed that the blade (18) is arranged in offset relation to a longitudinal plane of the handle (12), **characterized in that** the blade (18) forms a first electrode, and **in that** a second electrode (60) is arranged on the blade holder (14) in spaced relation to the blade (18).

2. Surgical peeling knife in accordance with claim 1, **characterized in that** with respect to respective side faces the blade (18) and the second electrode (60) are aligned substantially parallel to each other.

3. Surgical peeling knife in accordance with claim 1 or 2, **characterized in that** with respect to an end (34) of the blade holder (14) the second electrode (60) is located in front of the blade (18).

4. Surgical peeling knife in accordance with any one of the preceding claims, **characterized in that** the blade (18) is so arranged that a cutting edge (48) is orientated away from the handle (12).

5. Surgical peeling knife in accordance with any one of the preceding claims, **characterized in that** a cutting edge (48) of the blade (18) is at a greater distance with respect to height from the handle (12) than an upper edge of the second electrode (60).

6. Surgical peeling knife in accordance with any one of the preceding claims, **characterized in that** the blade (18) is so designed that its cutting edge (48) is seated below a crest (38) of the blade holder (14) in the direction of a front end (34) of the blade holder (14).

7. Surgical peeling knife in accordance with any one of the preceding claims, **characterized in that** the blade holder (14) comprises a first bracket (22) and a second bracket (24), between which the blade (18) and the second electrode (60) are arranged and between which a space (26) is formed.

8. Surgical peeling knife in accordance with any one of the preceding claims, **characterized in that** the peeling knife (10) is substantially made of a plastics material.

9. Surgical peeling knife in accordance with any one of the preceding claims, **characterized in that** an electrical lead (52, 62) to the electrodes (18, 60) is embedded in the handle (12) and/or the blade holder (14).

10. Surgical peeling knife in accordance with any one of the preceding claims, **characterized by** a high-frequency connection (56, 64) for the electrodes (18, 60).

11. Surgical peeling knife in accordance with any one of the preceding claims, **characterized in that** a blade receptacle (40) with one or a plurality of positive-locking elements for securing the blade (18) is arranged on the blade holder (14).

12. Surgical peeling knife in accordance with any one of the preceding claims, **characterized in that** a blade receptacle (40) is connected to an electrical lead (52) for supplying the blade (18) with power.

13. Surgical peeling knife in accordance with claim 12, **characterized in that** the blade receptacle (40) comprises a contact element (42) for the blade (18).

14. Surgical peeling knife in accordance with claim 13, **characterized in that** a conductive spring element (44) biased in the direction of the blade (18) is arranged on the contact element (42).

15. Surgical peeling knife in accordance with claim 13, **characterized in that** the contact element is fixedly connected to the blade (18).

## Revendications

1. Racloir ou rabot chirurgical comprenant une poignée de maintien (12) et, fixé à la poignée de maintien (12), un support de lame de coupe de coupe (14) maintenant une lame de coupe (18) métallique, qui est orientée transversalement par rapport à une direction longitudinale (36) de la poignée de maintien (12), le support de lame de coupe (14) étant conçu pour que la lame de coupe (18) soit agencée de façon décalée par rapport à un plan longitudinal de la poignée de maintien (12),
**caractérisé en ce que** la lame de coupe (18) forme une première électrode et **en ce qu'**à distance de la lame de coupe (18), sur le support de lame de coupe (14), est agencée une deuxième électrode (60).

2. Rabot chirurgical selon la revendication 1, **caractérisé en ce que** la lame de coupe (18) et la deuxième électrode (60) sont, relativement à leurs faces latérales, orientées sensiblement de manière parallèle l'une à l'autre.

3. Rabot chirurgical selon les revendications 1 ou 2, **caractérisé en ce que**, par rapport à une extrémité (34) du support de lame de coupe (14), la deuxième électrode (60) se situe avant la lame de coupe (18).

4. Rabot chirurgical selon l'une des revendications précédentes, **caractérisé en ce que** la lame de coupe (18) est agencée de façon à ce qu'un tranchant de coupe (48) soit orienté de manière à être éloigné de la poignée de maintien (12).

5. Rabot chirurgical selon l'une des revendications précédentes, **caractérisé en ce qu'**un tranchant de coupe (48) de la lame de coupe (18) présente une distance en hauteur plus grande par rapport à la poignée de maintien (12) qu'un bord supérieur de la deuxième électrode (60).

6. Rabot chirurgical selon l'une des revendications précédentes, **caractérisé en ce que** la lame de coupe (18) est conçue de façon à ce que son tranchant de coupe (48) soit situé en-dessous d'un sommet (38) du support de lame de coupe (14) en direction d'une extrémité avant (34) du support de lame de coupe (14).

7. Rabot chirurgical selon l'une des revendications précédentes, **caractérisé en ce que** le support de lame de coupe (14) comprend un premier étrier (22) et un deuxième étrier (24) entre lesquels sont agencées la lame de coupe (18) et la deuxième électrode (60), et entre lesquels est formé un espace intermédiaire (26).

8. Rabot chirurgical selon l'une des revendications précédentes, **caractérisé en ce que** le rabot (10) est fabriqué essentiellement en une matière plastique.

9. Rabot chirurgical selon l'une des revendications précédentes, **caractérisé en ce qu'**un système de raccordement électrique (52, 62) aux électrodes (18, 60) est noyé dans la poignée de maintien (12) et/ou le support de lame de coupe (14).

10. Rabot chirurgical selon l'une des revendications précédentes, **caractérisé par** un branchement de connexion haute fréquence (56, 64) pour les électrodes (18, 60).

11. Rabot chirurgical selon l'une des revendications précédentes, **caractérisé en ce que** sur le support de lame de coupe (14) est agencé un emplacement de réception de lame de coupe (40) avec un ou plusieurs éléments de liaison par complémentarité de forme pour la fixation de la lame de coupe (18).

12. Rabot chirurgical selon l'une des revendications précédentes, **caractérisé en ce qu'**un emplacement de réception de lame de coupe (40) est relié à un système de raccordement électrique (52) pour l'alimentation électrique de la lame de coupe (18).

13. Rabot chirurgical selon la revendication 12, **caractérisé en ce que** l'emplacement de réception de lame de coupe (40) présente un élément de contact (42) pour la lame de coupe (18).

14. Rabot chirurgical selon la revendication 13, **caractérisé en ce que** sur l'élément de contact (42) est agencé un élément conducteur (44) élastique, qui est précontraint en direction de la lame de coupe (18).

15. Rabot chirurgical selon la revendication 13, **caractérisé en ce que** l'élément de contact est relié de manière fixe à la lame de coupe (18).
